# EUROPEAN PATENT APPLICATION

(11) **EP 2 294 990 A2**
(43) Date of publication of application: **16.03.2011**
(21) Application number: 10153207.5
(22) Date of filing: 10.02.2010
(51) Int. Cl.: A61B 17/122, A61B 17/128

(54) **Hermostatic clip and hemostatic clip operation apparatus using the same**

(30) Priority: 14.09.2009 KR 20090086286
(71) Applicant: National Cancer Center, Ilsandong-gu Goyang-si, Gyeonggi-do (KR)
(72) Inventor: Cho, Seong-Yeon, Gyeonggi-do (KR); Park, Sang-Jae, Gyeonggi-do (KR); Kim, Seoung-Hoon, Gyeonggi-do (KR); Han, Sung-Sik, Gyeonggi-do (KR); Kim, Young-Kyu, Gyeonggi-do (KR); Kim, Kwang-Gi, Gyeonggi-do (KR); Kim, Hyung-Tae, Gyeonggi-do (KR); Kim, Hyun-Ho, Gyeonggi-do (KR)
(74) Representative: Schoppe, Fritz

(57) **Abstract**

Provided are a hemostatic clip and a hemostatic clip operation apparatus using the same that can rapidly stop bleeding from a bleeding area of a blood vessel when a middle or large diameter blood vessel partially bleeds during an abdominal operation or a surgery using an endoscope. The hemostatic clip operation apparatus includes a handle, a first gripper configured to primarily stop bleeding from a bleeding area of a blood vessel, a second gripper configured to receive the first gripper and symmetrically bend a closed-loop shaped-hemostatic clip to secondarily clip a periphery of the bleeding area stopped by the first gripper, and a gripper driver configured to connect the handle to the first and second grippers to be interlocked with operation of the handle to operate sequential stopping of bleeding and clipping of the first and second grippers.

## Description

### CROSS-REFERENCE TO RELATED APPLICATION

This application claims priority to and the benefit of Korean Patent Application No. 2009-86286, filed September 14, 2009, the disclosure of which is incorporated herein by reference in its entirety.

### BACKGROUND

### 1. Field of the Invention

The present invention relates to a hemostatic clip and a hemostatic clip operation apparatus using the same.

### 2. Discussion of Related Art

In general, since a middle or large diameter arterial blood vessel may cause a serious problem upon bleeding due to even a minor injury, it requires careful attention. During an abdominal operation or laparoscopy, the middle or large diameter blood vessel may bleed due to mistakes or accidents. At this time, a hemostatic clip and a hemostatic clip operation apparatus on which the hemostatic clip is mounted are used to stop bleeding from the surface of the blood vessel.

Conventional hemostatic clips are disclosed in US Patent No. 4,799,481, entitled "Surgical Hemostatic Clips,", US Patent No. 4,611,595, entitled "Spring Activated Hemostatic Clip Applicator," and US Patent No. 6,346,112, entitled "Hemostatic Clips."

However, when the conventional hemostatic clip is used to stop the bleeding from the middle or large diameter arterial blood vessel, the hemostatic clip may block the entire blood vessel as well as the bleeding area, causing serious problems in blood circulation. Therefore, when the middle or large diameter blood vessel partially bleeds, a novel hemostatic clip and a hemostatic clip operation apparatus that are capable of rapidly stopping bleeding in the bleeding area, without blocking the entire blood vessel, are needed.

### SUMMARY OF THE INVENTION

The present invention is directed to a hemostatic clip capable of rapidly stopping bleeding in a bleeding area, without blocking the entire blood vessel, when a middle or large blood vessel bleeds during an abdominal operation or an operation using an endoscope.

The present invention is also directed to a hemostatic clip operation apparatus using the hemostatic clip.

Additional aspects of the invention will be set forth in the description which follows, and in part will be apparent from the description, or may be learned by practice of the invention.

In an example embodiment, a hemostatic clip includes: a clip body configured to clip a periphery of a bleeding area of a blood vessel to stop bleeding; and a pair of bending parts formed at upper and lower sides of a symmetrical center line of the clip body to symmetrically bend the clip body with respect to the center line.

Here, the clip body may have a circular or polygonal closed-loop shape, more preferably, an oval closed-loop shape.

In addition, the bending parts may be disposed on a major axis of the clip body. The clip body may further include a pair of hook parts formed at the clip body to hook and remove the hemostatic clip clipped to the bleeding area.

Here, the hook parts may project from both left and right sides of the clip body opposite to the bleeding area.

In another example embodiment, a hemostatic clip operation apparatus includes: a handle; a first gripper configured to primarily stop bleeding from a bleeding area of a blood vessel; a second gripper configured to receive the first gripper and symmetrically bend a closed-loop shaped-hemostatic clip to secondarily clip a periphery of the bleeding area stopped by the first gripper; and a gripper driver configured to connect the handle to the first and second grippers to be interlocked with operation of the handle to operate sequential stopping of bleeding and clipping of the first and second grippers.

Here, the handle may include a handle body, and a pair of operation handles hinged to both sides of the handle body to be pivoted inward and outward.

In addition, the gripper driver may include: a push rod movably inserted into the handle body forward and backward; a first link member configured to link the operation handle to the push rod to guide reciprocal movement of the push rod depending on pivotal movement of the operation handle; a first operation rod disposed in front of the push rod, and configured to move forward by the push rod to perform a bleeding-stopping operation of the first gripper; a second operation rod disposed in front of the push rod to receive the first operation rod and move forward by the push rod to perform a clipping operation of the second gripper when the bleeding-stopping operation of the first gripper is completed; a second link member inserted to pass through the center of the push rod and the first operation rod and linked to the first and second grippers at its tip; and a guide case coupled to a tip of the handle body and configured to guide such that a head of the push rod and the second operation rod are inserted into the guide case to move straightly.

In addition, the push rod may have a rear end hinged to the first link member to move forward by the first link member when the pair of operation handles are pivoted inward, and move backward by the first link member when the operation handles are pivoted outward.

Further, a resilient member may be provided between the push rod and the first operation rod.

Here, the resilient member may include a spring configured to push the first operation rod forward by the push rod in a non-compressed state when the pair of operation handles are pivoted inward halfway, and to be compressed not to push the first operation rod forward when the operation handles are pivoted inward completely.

Furthermore, when the pair of operation handles are pivoted inward halfway, the resilient member may push the first operation rod using a force of the push rod moving forward, and the first operation rod may move forward to press an outer surface of the first gripper to close the first gripper.

In addition, when the operation handle is pivoted inward completely, the resilient member may be compressed to move the push rod forward to push the second operation rod, and the second operation rod may be moved forward to press an outer surface of the second gripper to close the second gripper.

Further, when the first gripper is completely closed, the first gripper may have an outer width that gradually increases toward the bleeding area, and the first operation rod may have an inner width smaller than the largest outer width of the first gripper, so that the first operation rod cannot move forward.

In addition, instead of omission of the resilient member provided between the push rod and the first operation rod, the operation handle and the hinge coupling part of the first link member may be configured to slide forward and backward upon pivotal movement of the operation handle.

Further, the front end of the second operation rod opposite to the second gripper may have a V-shaped groove.

Furthermore, the first gripper may include a pair of gripper members connected to a tip of the second link member to be laterally opened and closed.

In addition, the second gripper may include a pair of gripper members connected to the second link member in the rear of the first gripper to be laterally opened and closed while receiving the first gripper.

Further, the second gripper may have a first gripper receiving groove at a front surface opposite to the first gripper to receive the first gripper when the second gripper is laterally closed.

Furthermore, the second gripper may include clip holding parts formed at left and right ends thereof to hold left and right sides of the hemostatic clip, and clip bending parts formed at upper and lower ends thereof to press and bend upper and lower sides of the hemostatic clip.

Specific descriptions of other example embodiments will be apparent from the detailed description and the attached drawings.

### BRIEF DESCRIPTION OF THE DRAWINGS

The above and other objects, features and advantages of the present invention will become more apparent to those of ordinary skill in the art by describing in detail example embodiments thereof with reference to the attached drawings, in which:
FIG. 1 is a perspective view of a hemostatic clip in accordance with an example embodiment of the present invention;
FIG 2 is a plan view of the hemostatic clip shown in FIG. 1;
FIG. 3 is a plan view of a hemostatic clip having a polygonal shape;
FIG. 4 is a perspective view of a hemostatic clip operation apparatus using a hemostatic clip in accordance with an example embodiment of the present invention;
FIG. 5 is an exploded perspective view of the hemostatic clip operation apparatus shown in FIG. 4;
FIG. 6 is an enlarged perspective view of portion A of FIG. 4;
FIG. 7 is an enlarged perspective view of portion B of FIG. 4;
FIG. 8 is a perspective view of an example embodiment of portion C of FIG. 4;
FIG. 9 is a perspective view of another example embodiment of portion C of FIG. 4;
FIG. 10 is a view showing the hemostatic clip operation apparatus holding the hemostatic clip in accordance with the present invention;
FIG. 11 is an enlarged cross-sectional view of portion D of FIG. 10;
FIG. 12 is an enlarged perspective view of portion E of FIG. 10;
FIG. 13 is a view for explaining a bleeding-stopping operation of a first gripper according to a first operation of the hemostatic clip operation apparatus in accordance with the present invention;
FIG. 14 is an enlarged cross-sectional view of portion F of FIG. 13;
FIG. 15 is an enlarged perspective view of portion G of FIG. 13;
FIG. 16 is a view for explaining a clipping operation of a second gripper according to a second operation of the hemostatic clip operation apparatus in accordance with the present invention;
FIG. 17 is an enlarged cross-sectional view of portion H of FIG. 16;
FIG. 18 is an enlarged perspective view of portion I of FIG. 16;
FIGS. 19 and 20 are views showing a state in which the hemostatic clip clips a bleeding area of a blood vessel using the hemostatic clip operation apparatus in accordance with the present invention;
FIG. 21 is a view showing that the hemostatic clip in accordance with the present invention is removed; and
FIG. 22 is an enlarged perspective view of portion J of FIG. 21.

### DETAILED DESCRIPTION OF EXAMPLE EMBODIMENTS

Example embodiments of the present invention will be described in detail. However, the present invention is not limited to the embodiments disclosed below, but can be implemented in various forms. The following embodiments are described in order to enable those of ordinary skill in the art to embody and practice the present invention. Like reference numerals designate like elements throughout the detailed description.

Hereinafter, a hemostatic clip and a hemostatic clip operation apparatus using the same in accordance with an example embodiment of the present invention will be described with reference to the accompanying drawings. In the detailed description, if it is determined that description of conventional functions or constitutions may make the sprit of the invention unclear, detailed description thereof will be omitted.

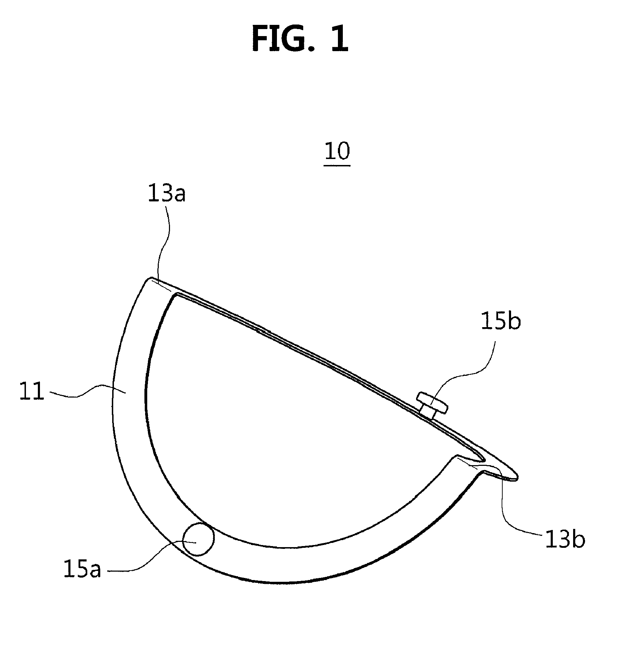

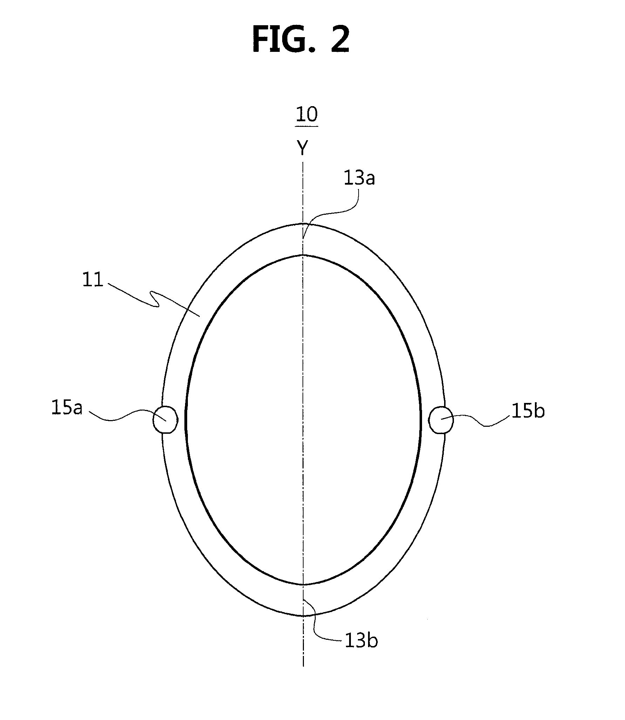

FIG. 1 is a perspective view of a hemostatic clip in accordance with an example embodiment of the present invention, and FIG. 2 is a plan view of the hemostatic clip shown in FIG. 1.

As shown in FIGS. 1 and 2, a hemostatic clip 10 in accordance with an example embodiment of the present invention may include a clip body 11, bending parts 13a and 13b, and hook parts 15a and 15b.

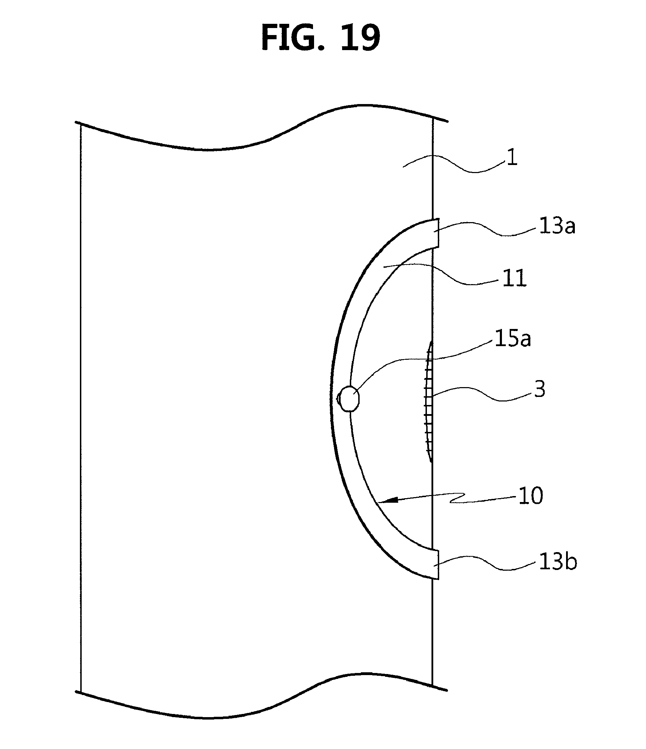

The clip body 11 functions to clip a periphery of a bleeding area 3 of an important blood vessel 1 such as a middle or large diameter blood vessel (see FIG. 19), while being held by a second gripper 220 of a hemostatic clip operation apparatus 20, which will be described below.

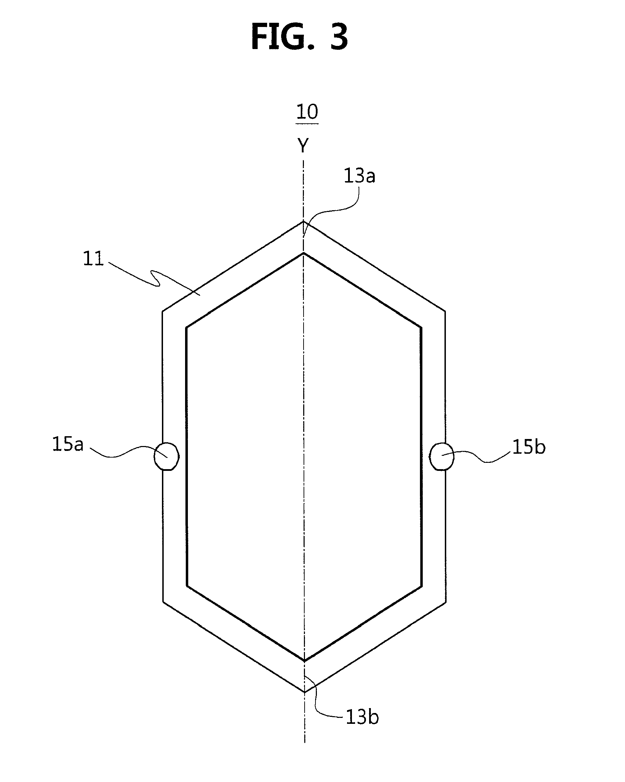

The clip body 11 may have a symmetrical circular closed loop shape. While the example embodiment illustrates the clip body 11 having a circular shape, the clip body 11 may have a polygonal closed loop shape such as a symmetrical rectangular shape (not shown), a hexagonal shape (see FIG. 3), etc., not limited to the circular shape. Here, the clip body 11 may have a vertical length larger than a lateral length when the clip body 11 is symmetrical with respect to a line connecting the bending parts 13a and 13b as a centerline to minimize a hemostatic range for the diameter of the blood vessel 1. At this time, the bending parts 13a and 13b are disposed on a major shaft Y of the clip body 11.

In addition, the clip body 11 may be formed of a material such as a biocompatible metal.

The pair of bending parts 13a and 13b may be formed at upper and lower sides of the clip body 11 with respect to a centerline thereof such that the clip body 11 is symmetrically bent with respect to the centerline.

The bending parts 13a and 13b may have a predetermined elasticity such that the bending parts 13a and 13b can be pressed and bent by clip bending parts 220c and 220d of a second gripper 220, which will be described below.

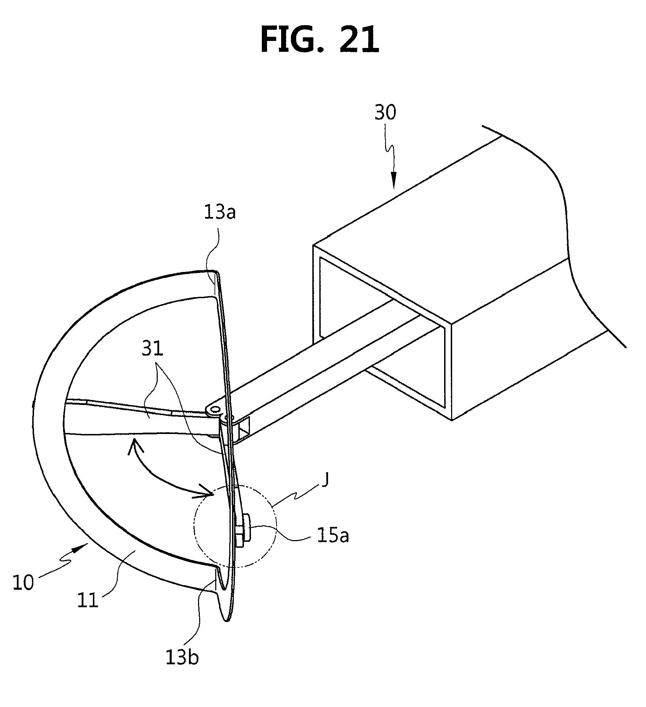

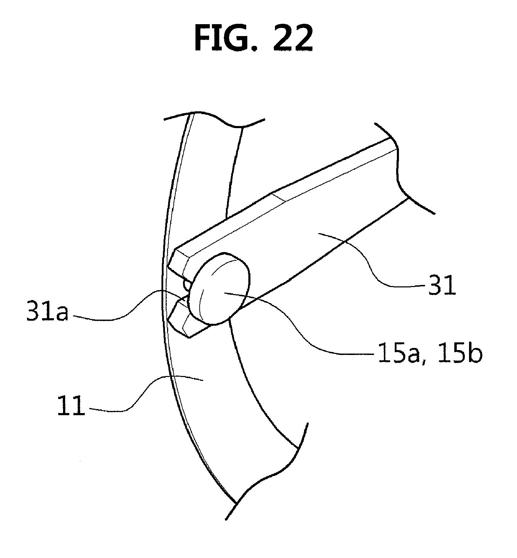

The pair of hook parts 15a and 15b may be formed at the clip body 11 such that the hemostatic clip 10 clipped at the bleeding area 3 of the blood vessel 1 can be easily removed using a separate clip removing device 30 (see FIGS. 21 and 22).

The hook parts 15a and 15b may project from both sides of the clip body 11 opposite to the bleeding area 3. At this time, the hook parts 15a and 15b may project from both sides of the clip body 11 in a ring shape, and may have a diameter of a head larger than that of an extension part. In the clip removing device 31, fastening members 31 fastened to the hook part 15a and 15b may have fastening grooves 31a which are formed at an end and widen from a rear part to a front part, such that the fastening members 31 of the clip removing device 30 can be readily fastened to the hook parts 15a and 15b of the hemostatic clip 10.

In addition, the hook parts 15a and 15b are configured to be easily fastened to the clip removing device 30, and to substantially endure an external force pulling out to widen the hemostatic clip 10, removing the hemostatic clip 10 held therein.

While the example embodiment illustrates that the hook parts 15a and 15b are separately coupled to the clip body 11, the hook parts 15a and 15b may be integrally formed with the clip body 11, not limited thereto.

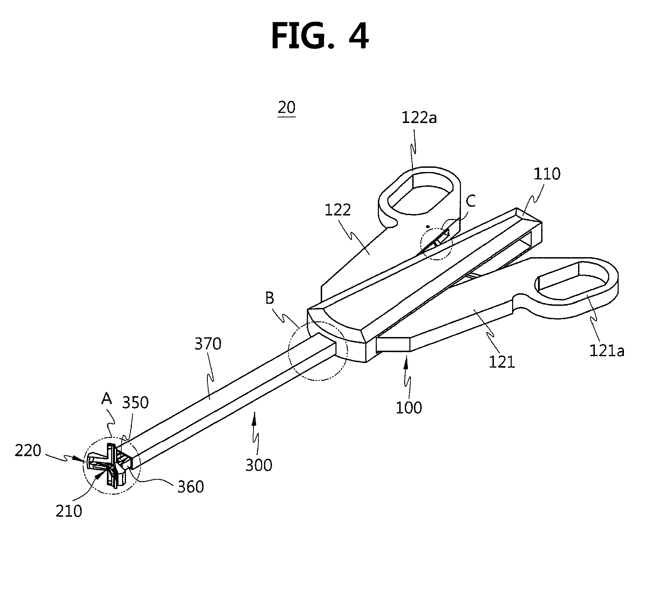

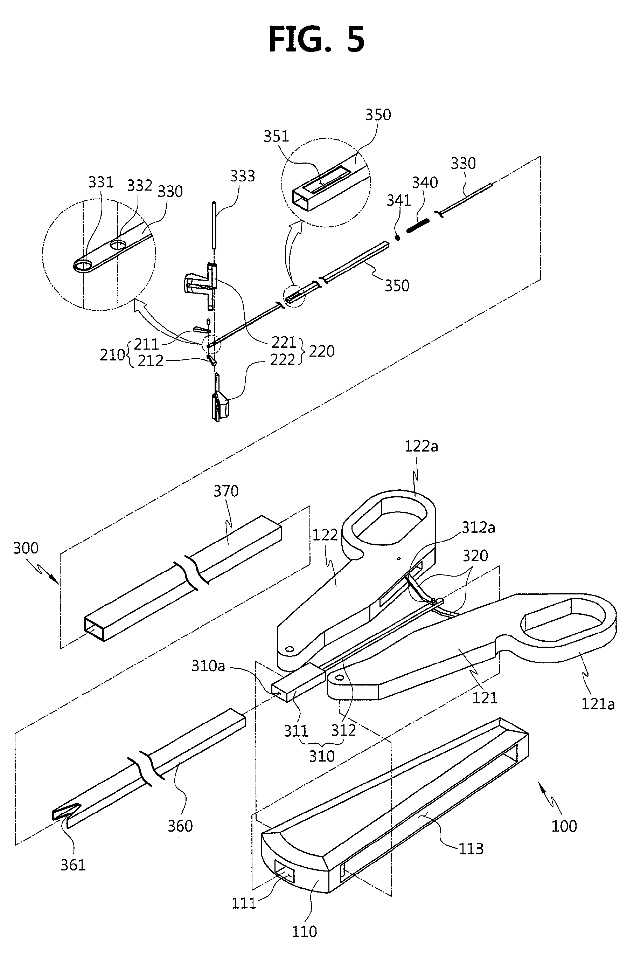

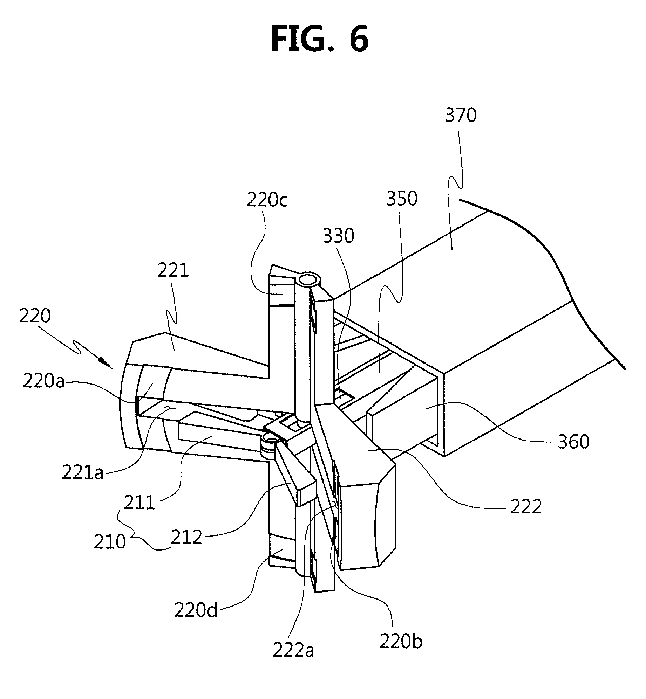

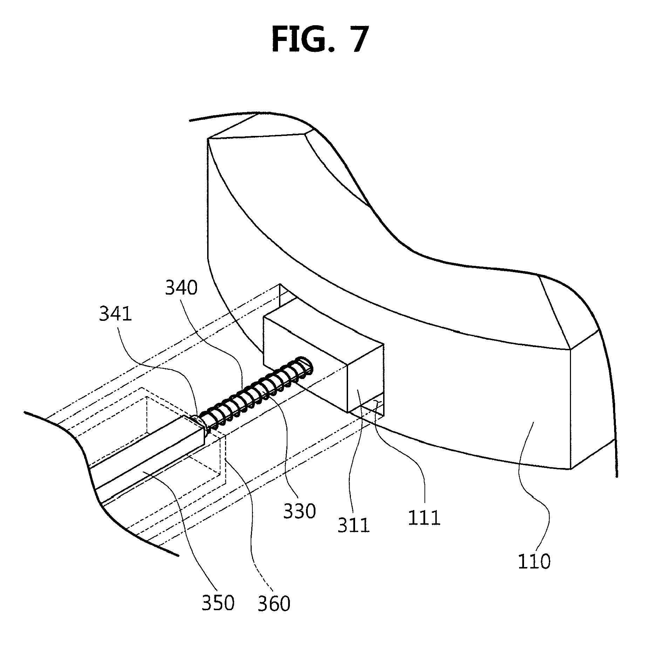

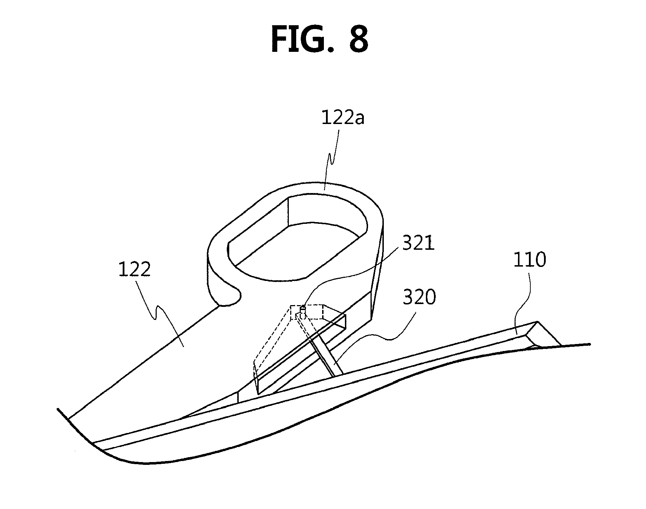

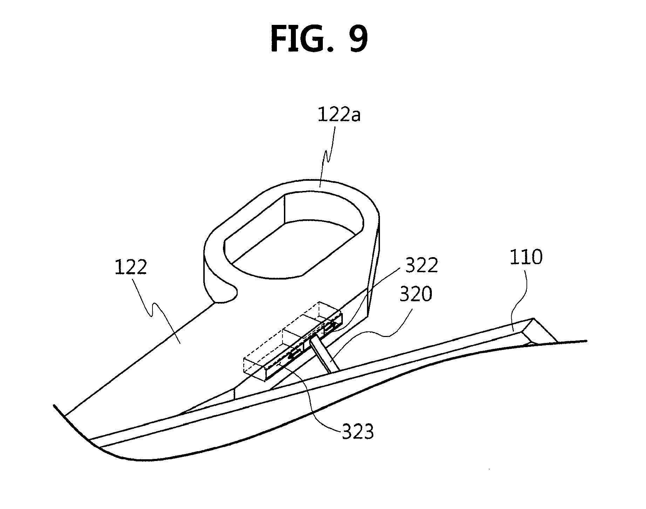

FIG. 4 is a perspective view of a hemostatic clip operation apparatus using a hemostatic clip in accordance with an example embodiment of the present invention, FIG. 5 is an exploded perspective view of the hemostatic clip operation apparatus shown in FIG. 4, FIGS. 6 and 7 are enlarged perspective views of portions A and B of FIG 4, respectively, and FIGS. 8 and 9 are perspective views of example embodiments of portion C of FIG. 4.

As shown in FIGS. 4 to 9, the hemostatic clip operation apparatus 20 in accordance with an example embodiment of the present invention may include a handle 100, a first gripper 210, a second gripper 220, and a gripper driver 300.

The handle 100 functions to directly apply an external force to the gripper driver 300 to perform a bleeding-stopping and clipping operation of the first and second grippers 210 and 220 in a state in which a surgeon holds the handle 100.

The handle 100 may include a handle body 110, and a pair of operation handles 121 and 122 pivotally hinged inward and outward at both sides of the handle body 110. Here, the handle body 110 may have a hole 111 formed at a tip thereof and through which a push rod 310, a second link member 330 and a guide case 370 are inserted, and open surfaces 113 formed at both sides thereof such that the pair of operation handles 121 and 122 are pivoted inward to be received therein. In addition, front ends of the operation handles 121 and 122 are hinged to the handle body 110, and rear ends of the operation handles 121 and 122 are linked to the push rod 310 by the first link member 320, which will be described below. Further, the rear ends of the operation handles 121 and 122 may have finger insertion parts 121a and 122a into which a surgeon can insert his/her fingers to hold them.

The first gripper 210 functions to primarily stop bleeding from the bleeding area 3 of the blood vessel 1. More specifically, when the pair of operation handles 121 and 122 are pivoted inward halfway, a first operation rod 350 moves forward to press an outer surface of the first gripper 210 so that the first gripper 210 closes the bleeding area 3 to primarily stop bleeding.

The first gripper 210 may be constituted by a pair of gripper members 211 and 212 connected to the tip of the second link member 330 to be laterally opened and closed. Here, when the first gripper 210 is completely closed, the first gripper 210 may have an outer width that gradually increases toward the bleeding area such that the first operation rod 350 cannot move forward, and the first operation rod 350 may have an inner width smaller than the largest outer width of the first gripper 210.

The second gripper 220 functions to symmetrically bend the closed-loop shaped hemostatic clip 10 shown in FIGS. 1 and 2 and secondarily clip a periphery of the bleeding area 3 stopped by the first gripper 210. More specifically, when the pair of operation handles 121 and 122 are pivoted inward completely, a second operation rod 360 moves forward to pressure the periphery of the second gripper 220 so that the second gripper 220 is closed to secondarily clip the periphery of the bleeding area 3.

The second gripper 220 may be constituted by a pair of gripper members 221 and 222 connected to receive the first gripper 210 in the second link member 330 in the rear of the first gripper 210 and to be laterally opened and closed. While this example embodiment illustrates a constitution in which the pair of gripper members 221 and 222 are symmetrically coupled to form a "+" shape and laterally opened and closed with respect to a center cross line, the second gripper 220 may have various constitutions, not limited thereto.

The second gripper 220 is configured to receive the first gripper 210. For example, the second gripper 220 may have first gripper receiving grooves 221a and 222a larger than the first gripper 210 and opposite to the first gripper 210 so that the first gripper 210 is accommodated in the second gripper 220 upon closing of the second gripper 220.

The second gripper 220 may include clip holding parts 220a and 220b formed at left and right ends thereof to hold left and right sides of the hemostatic clip 10, and clip bending parts 220c and 220d formed at upper and lower ends thereof to press and bend upper and lower sides of the hemostatic clip 10. Here, the clip holding parts 220a and 220b and the clip bending parts 220c and 220d may be formed at the second gripper 220 to form a stepped groove to prevent separation of the hemostatic clip 10 from the held state.

The gripper driver 300 functions to drive a sequential stopping of bleeding and clipping operation of the first and second grippers 210 and 220 according to a moving operation of the handle 100.

The gripper driver 300 is configured to connect the handle 100 to the first and second grippers 210 and 220. For this purpose, the gripper driver 300 may include a push rod 310, a first link member 320, a second link member 330, a first operation rod 350, a second operation rod 360, and a guide case 370.

The push rod 310 is disposed in the handle body 110 and a head 311 of the push rod 310 is movably inserted through a hole 111 formed at the tip of the handle body 110 forward and backward.

The push rod 310 may include the head 311, and a rod extension part 312 extending rearward from the head 311.

Here, contact areas of the head 311 of the push rod 310 and the second operation rod 360 may be equal to each other so that the head 311 of the push rod 310 pushes the second operation rod 360 forward when the pair of operation handles 121 and 122 are pivoted inward completely.

The rod extension part 312 of the push rod 310 may be connected to the first link member 320 by a hinge 312a such that the push rod 310 moves forward by the first link member 320 when the pair of operation handles 121 and 122 are pivoted inward and the push rod 310 moves backward by the first link member 320 when the operation handles 121 and 122 are pivoted outward.

In addition, the push rod 310 may have a hole 310a through which the second link member 330 is inserted forward and backward.

The first link member 320 links the operation handles 121 and 122 to the push rod 310 to guide reciprocal movement of the push rod 310 depending on pivotal movement of the operation handles 121 and 122.

When a spring 340 is installed between the push rod 310 and the first operation rod 350, as shown in FIG. 8, the operation handles 121 and 122 are hinged to the first link member 320, and a hinge coupling part 321 may be fixed to the operation handle 121 and 122. Therefore, when the pair of operation handles 121 and 122 are pivoted inward completely, the push rod 310 moves forward to push the second operation rod 360 so that the spring 340 is compressed not to push the first operation rod 350.

On the other hand, when the spring 340 is not installed between the push rod 310 and the first operation rod 350, as shown in FIG. 9, the operation handles 121 and 122 are hinged to the first link member 320, and the hinge coupling part 322 is inserted into slide guide grooves 323 formed in inner surfaces of the operation handles 121 and 122 to be slid forward and backward upon pivotal movement of the operation handles 121 and 122. Therefore, when the pair of operation handles 121 and 122 are pivoted inward completely, the push rod 310 moves forward to push the second operation rod 360 so that the hinge coupling part 322 moves backward along the slide guide groove 323 not to push the first operation rod 350.

The second link member 330 has a long-plated rod shape, and is inserted to pass through the push rod 310, the spring 340, and the first operation rod 350. The second link member 330 may have a length larger than the entire straight length of the push rod 310, the spring 340 and the first operation rod 350. The second link member 330 may have two link holes 331 and 332 formed at its tip to be linked to the first and second grippers 210 and 220, respectively.

The first operation rod 350 is disposed in front of the push rod 310, and functions to move forward by the push rod 310 to make the first gripper 210 perform the stopping of bleeding.

The first operation rod 350 may have a rectangular or circular hollow rod shape through which the second link member 330 can be inserted. In addition, the first operation rod 350 has an elongated hole 351 formed at its tip so that the second gripper 220 is coupled to a link pin 333 inserted through the link hole 332 of the second link member 330 and the elongated hole 351 of the first operation rod 350.

In addition, a resilient member 340 may be installed between the push rod 310 and the first operation rod 350. Here, a sealing member 341 is disposed at a rear end of the first operation rod 350 to be in contact with a tip of the resilient member 340. Here, the resilient member 340 may be a spring (hereinafter, referred to by reference numeral 340) having a predetermined elasticity such that the push rod 310 moves forward to push the first operation rod 350 forward when the pair of operation handles 121 and 122 are pivoted inward halfway in a state in which the spring 340 is not compressed, and the first operation rod 350 is not pushed forward anymore when the operation handles 121 and 122 are pivoted inward completely to compress the spring 340. Therefore, when the pair of operation handles 121 and 122 are pivoted inward halfway, the push rod 310 moves forward to make the spring 340 push the first operation rod 350, and the first operation rod 350 moves forward to press a rear part of the periphery of the first gripper 210 forward, closing the first gripper 210. In addition, when the operation handles 121 and 122 are pivoted inward completely, the spring 340 is compressed to move the push rod 310 forward to push the second operation rod 360, and thus, the second operation rod 360 moves forward to press a rear part of the periphery of the second gripper 220 forward, closing the second gripper 220.

The second operation rod 360 is disposed in front of the push rod 310 to receive the first operation rod 350 therein. The second operation rod 360 functions to perform a clipping operation of the second gripper 220 by a forward-moving force of the push rod 310 when the stopping of bleeding by the first gripper 210 is completed.

The second operation rod 360 may have a rectangular or circular hollow rod shape such that the first operation rod 350 is inserted. In addition, contact areas of the rear end of the second operation rod 360 and the head 311 of the push rod 310 may be equal to each other such that the head 311 of the push rod 310 can push the second operation rod 360 forward when the operation handles 121 and 122 are pivoted inward completely.

Further, the second operation rod 360 may have a V-shaped groove 361 formed at its tip opposite to the second gripper 220 such that the second operation rod 360 moves forward to press the periphery of the second gripper 220 to close the second gripper 220 and at the same time receive the rear end of the closed second gripper 220. While this example embodiment illustrates the second gripper 220 having the V-shaped groove formed at its tip, the second gripper 220 may have a U-shaped groove, not limited thereto.

The guide case 370 is inserted into the hole 111 formed at the tip of the handle body 110 to be coupled thereto such that the head 311 of the push rod 310 and the second operation rod 360 are inserted to be guided straightly. Here, the guide case 370 has an inner diameter substantially the same as or slightly larger than outer diameters of the head 311 of the push rod 310 and the second operation rod 360.

While this example embodiment illustrates the guide case 370 having a rectangular hollow rod shape, the guide case 370 may have a circular hollow rod shape, not limited thereto.

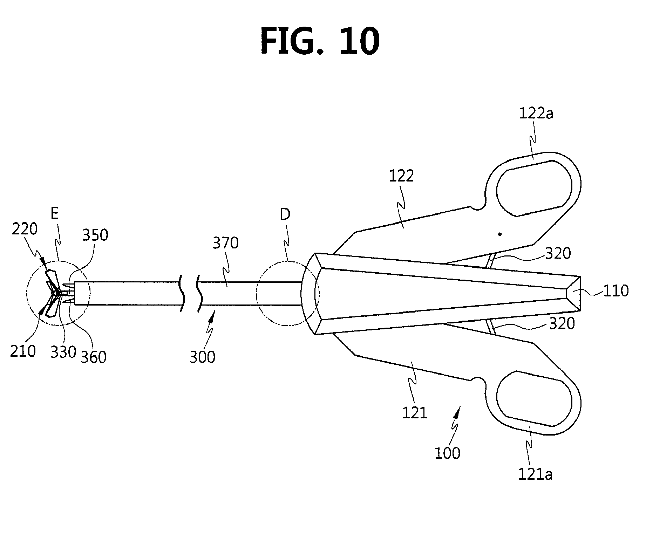

Hereinafter, operations of the hemostatic clip operation apparatus using the hemostatic clip in accordance with the present invention will be described in detail with reference to FIGS. 10 to 18.

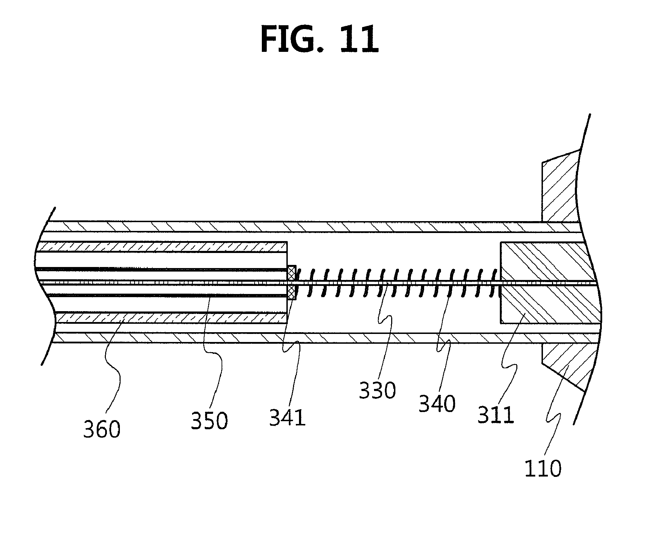

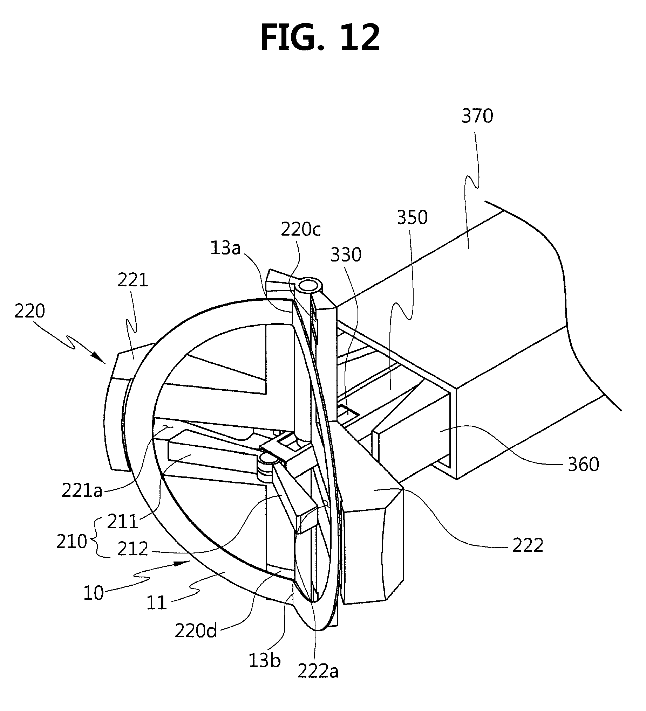

FIG. 10 is a view showing the hemostatic clip operation apparatus holding the hemostatic clip in accordance with the present invention, FIG. 11 is an enlarged cross-sectional view of portion D of FIG. 10, and FIG. 12 is an enlarged perspective view of portion E of FIG. 10.

As shown in FIGS. 10 to 12, the hemostatic clip 10 in accordance with the present invention may be mounted in the hemostatic clip operation apparatus 20 and used therewith. The operation handles 121 and 122 of the handle 100 of the hemostatic clip operation apparatus 20 are pivoted outward to hold the hemostatic clip 10 to the second gripper 220 in a state in which both the first gripper 210 and the second gripper 220 are widened. That is, the first gripper 210 and the second gripper 220 maintain the widened state in a state in which the hemostatic clip 10 is mounted in the hemostatic clip operation apparatus 20. At this time, the clip holding parts 220a and 220b formed at left and right ends of the second gripper 220 hold left and right sides of the clip body 11, respectively, and the clip bending parts 220c and 220d formed at upper and lower ends of the second gripper 220 press upper and lower sides of the clip body 11 to bend the clip body 11. In addition, the first gripper 210 is disposed inside the widened second gripper 220 and directed to a center open surface of the clip body 11.

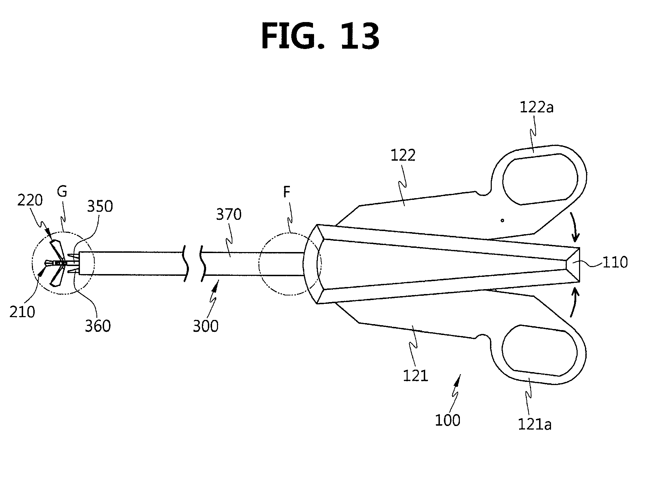

When a middle or large diameter blood vessel such as an arterial blood vessel 1 partially bleeds, a surgeon can sequentially operate the first and second grippers 210 and 220 to make the hemostatic clip 10 clip the bleeding area 3 to stop the bleeding through a stepped gripping operation of the handle 100 of the hemostatic clip operation apparatus 20. The clipping operation of the bleeding area 3 according to the sequential operation of the hemostatic clip operation apparatus 20 will be described in detail with reference to FIGS. 13 to 18.

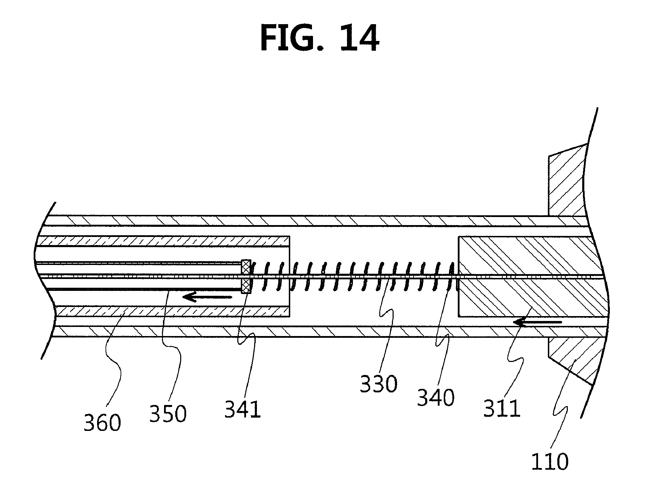

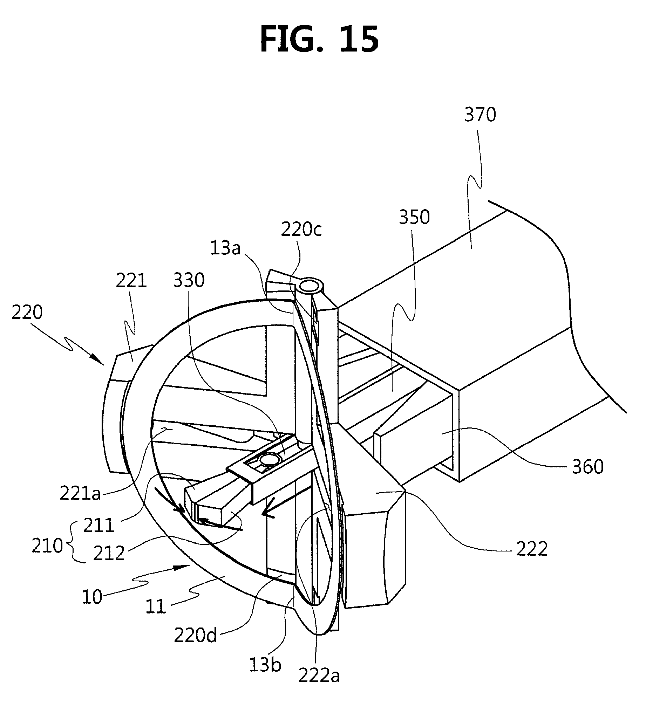

FIG. 13 is a view for explaining a bleeding-stopping operation of a first gripper according to a first operation of the hemostatic clip operation apparatus in accordance with the present invention, FIG. 14 is an enlarged cross-sectional view of portion F of FIG. 13, and FIG. 15 is an enlarged perspective view of portion G of FIG. 13.

As shown in FIGS. 13 to 15, when a surgeon grips the handle 100 of the hemostatic clip operation apparatus 20 inward halfway, the first gripper 210 is closed to primarily stop bleeding from the bleeding area 3 of the blood vessel 1. More specifically, when the pair of operation handles 121 and 122 of the handle 100 are pivoted inward halfway, a force applied by the handle 100 is transmitted through the first link member 320 connected to the operation handles 121 and 122 to move the push rod 310 forward. As the push rod 310 moves forward, the spring 340 in front of the push rod 310 first pushes the first operation rod 350, and the first operation rod 350 moves forward to press the periphery of the first gripper 210 to close the first gripper 210, primarily stopping the bleeding from the bleeding area 3. Here, since the first gripper 210 has an outer width that gradually increases toward the bleeding area 3 and the first operation rod 350 has an inner width smaller than the largest outer width of the first gripper 210, when the first gripper 210 is completely closed, the first operation rod 350 can no longer move forward.

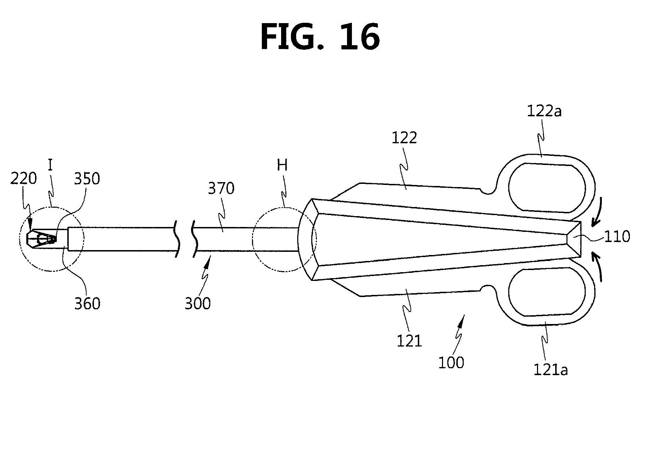

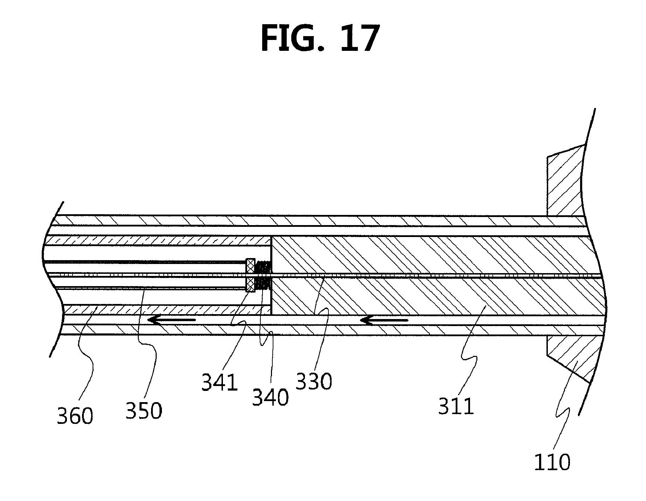

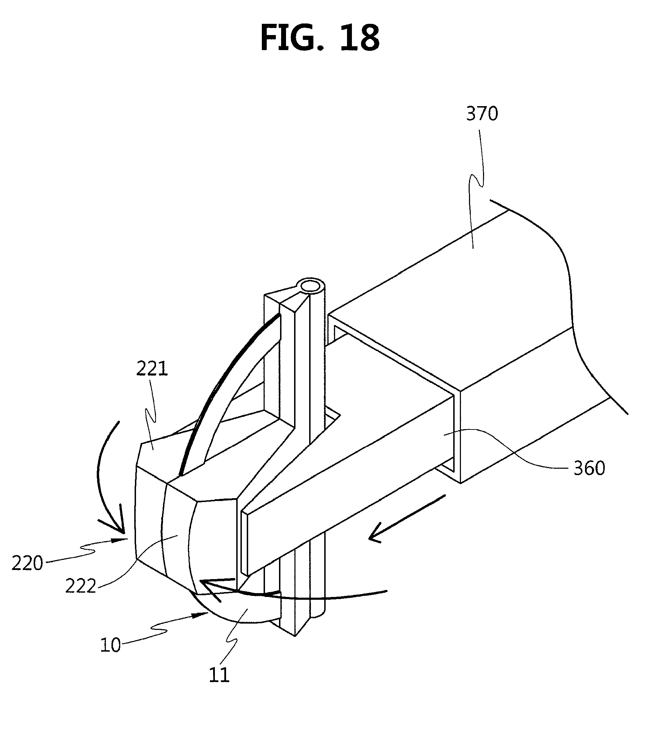

FIG. 16 is a view for explaining a clipping operation of a second gripper according to a second operation of the hemostatic clip operation apparatus in accordance with the present invention, FIG. 17 is an enlarged cross-sectional view of portion H of FIG. 16, and FIG. 18 is an enlarged perspective view of portion I of FIG. 16.

As shown in FIGS. 16 to 18, when the first gripper 210 is completely closed to primarily stop the bleeding from the bleeding area 3 and continuously apply a force to move the handle 100 inward completely, the second gripper 220 bends the hemostatic clip 10 to secondarily clip the periphery of the bleeding area 3. More specifically, when the pair of operation handles 121 and 122 are pivoted inward completely in a state in which the first gripper 210 stops the bleeding from the bleeding area 3, since the first operation rod 350 can no longer move forward, the spring 340 pressing the first operation rod 350 is compressed to move the push rod 310 forward to push the second operation rod 360 forward. Here, since the second operation rod 360 has a V- or U-shaped groove 361 formed at its surface in contact with the second gripper 220, the second operation rod 360 moves forward to press the rear part of the periphery of the second gripper 220 forward, and thus, the second gripper 220 is closed to symmetrically bend the hemostatic clip 10, secondarily stopping the bleeding from the bleeding area 3.

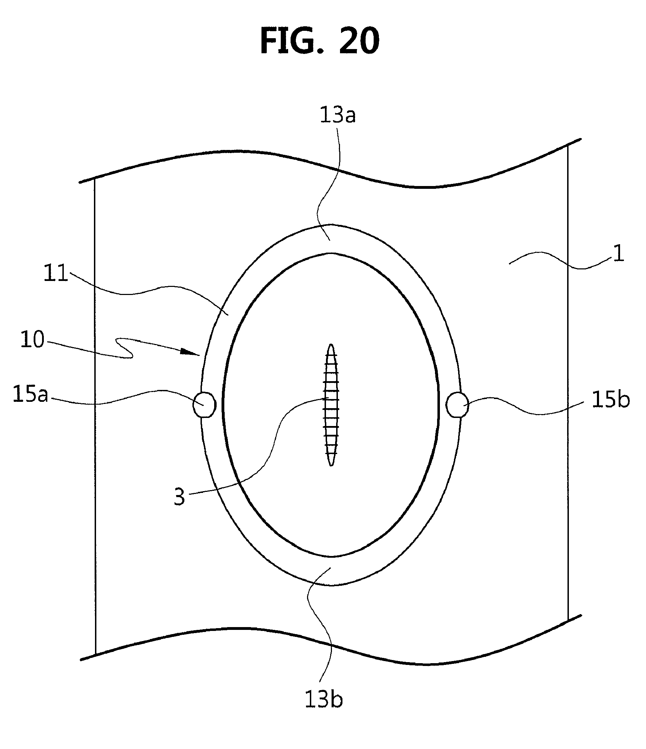

FIGS. 19 and 20 are views showing a state in which the hemostatic clip clips the bleeding area of the blood vessel using the hemostatic clip operation apparatus in accordance with the present invention.

As shown in FIGS. 19 and 20, when a middle or large diameter blood vessel such as the arterial blood vessel 1 partially bleeds, only the bleeding area 3 is partially clipped to stop the bleeding, without blocking the entire blood vessel, maintaining the function of the blood vessel 1. Therefore, the hemostatic clip 10 and the hemostatic clip operation apparatus 20 using the same in accordance with the present invention are appropriate to stop bleeding from the middle or large diameter blood vessel 1, the entire blocking of which may cause serious problems, upon bleeding.

As described above, the hemostatic clip operation apparatus 20 in accordance with the present invention has a multi-structure of the first and second grippers 210 and 220 having the function of precisely clipping the hemostatic clip 10 at the bleeding area, in addition to the function of simply clipping the hemostatic clip 10. In addition, the hemostatic clip 10 in accordance with the present invention also has a function of removing the clipped hemostatic clip 10 using the clip removing device 30, in addition to the function of clipping the bleeding area 3 of the blood vessel 1 to stop bleeding.

As can be seen from the foregoing, a hemostatic clip operation apparatus in accordance with the present invention can perform a function of precisely clipping a hemostatic clip at a bleeding area, in addition to a function of simply clipping the hemostatic clip using a multi-structure of first and second grippers.

In addition, a surgeon can easily stop bleeding from a blood vessel using the hemostatic clip by sequentially operating the first and second grippers through a step-by-step gripping operation of a handle of the hemostatic clip operation apparatus.

Further, when a middle or large diameter blood vessel such as an arterial blood vessel partially bleeds, it is possible to maintain the function of the blood vessel by partially clipping the bleeding area only, without blocking the entire blood vessel. Therefore, the hemostatic clip and the hemostatic clip operation apparatus using the same in accordance with the present invention are appropriate to stop bleeding from the middle or large diameter blood vessel, the entire blocking of which may cause serious problems, upon bleeding.

Furthermore, the hemostatic clip in accordance with the present invention can be readily removed when clipped to the blood vessel using a clip removing device, in addition to the function of clipping the bleeding area of the blood vessel.

While the invention has been shown and described with reference to certain example embodiments thereof, it will be understood by those skilled in the art that various changes in form and details may be made therein without departing from the spirit and scope of the invention as defined by the appended claims.

## Claims

1. A hemostatic clip operation apparatus comprising:
a handle;
a first gripper configured to primarily stop bleeding from a bleeding area of a blood vessel;
a second gripper configured to receive the first gripper and symmetrically bend a closed-loop shaped-hemostatic clip to secondarily clip a periphery of the bleeding area stopped by the first gripper; and
a gripper driver configured to connect the handle to the first and second grippers to be interlocked with operation of the handle to operate sequential stopping of bleeding and clipping of the first and second grippers.

2. The hemostatic clip operation apparatus according to claim 1, wherein the handle comprises:
a handle body; and
a pair of operation handles hinged to both sides of the handle body to be pivoted inward and outward.

3. The hemostatic clip operation apparatus according to claim 2, wherein the gripper driver comprises:
a push rod movably inserted into the handle body forward and backward;
a first link member configured to link the operation handle to the push rod to guide reciprocal movement of the push rod depending on pivotal movement of the operation handle;
a first operation rod disposed in front of the push rod, and configured to move forward by the push rod to perform a bleeding-stopping operation of the first gripper;
a second operation rod disposed in front of the push rod to receive the first operation rod and move forward by the push rod to perform a clipping operation of the second gripper when the bleeding-stopping operation of the first gripper is completed; and
a second link member inserted to pass through the center of the push rod and the first operation rod and linked to the first and second grippers at its tip.

4. The hemostatic clip operation apparatus according to claim 3, wherein the push rod has a rear end hinged to the first link member to move forward by the first link member when the pair of operation handles are pivoted inward, and move backward by the first link member when the operation handles are pivoted outward.

5. The hemostatic clip operation apparatus according to claim 3, further comprising a resilient member provided between the push rod and the first operation rod.

6. The hemostatic clip operation apparatus according to claim 5, wherein the resilient member comprises a spring configured to push the first operation rod forward by the push rod in a non-compressed state when the pair of operation handles are pivoted inward halfway, and to be compressed not to push the first operation rod forward when the operation handles are pivoted inward completely.

7. The hemostatic clip operation apparatus according to claim 5, wherein, when the pair of operation handles are pivoted inward halfway, the resilient member pushes the first operation rod using a force of the push rod moving forward, and the first operation rod moves forward to press an outer surface of the first gripper to close the first gripper.

8. The hemostatic clip operation apparatus according to claim 7, wherein, when the operation handles are pivoted inward completely, the resilient member is compressed to move the push rod forward to push the second operation rod, and the second operation rod is moved forward to press an outer surface of the second gripper to close the second gripper.

9. The hemostatic clip operation apparatus according to claim 3, wherein, when the first gripper is completely closed, the first gripper has an outer width that gradually increases toward the bleeding area, and the first operation rod has an inner width smaller than the largest outer width of the first gripper, so that the first operation rod cannot move forward.

10. The hemostatic clip operation apparatus according to claim 3, wherein the operation handle and the first link member are hinged to each other, and a hinge coupling part slides forward and backward upon pivotal movement of the operation handle.

11. The hemostatic clip operation apparatus according to claim 3, wherein the second rod has a groove formed at its tip opposite to the second gripper.

12. The hemostatic clip operation apparatus according to claim 3, wherein the gripper driver further comprises a guide case coupled to a tip of the handle body and configured to guide such that a head of the push rod and the second operation rod are inserted into the guide case to move straightly.

13. The hemostatic clip operation apparatus according to claim 3, wherein the first gripper includes a pair of gripper members connected to a tip of the second link member to be laterally opened and closed.

14. The hemostatic clip operation apparatus according to claim 13, wherein the second gripper includes a pair of gripper members connected to the second link member in the rear of the first gripper to be laterally opened and closed while receiving the first gripper.

15. The hemostatic clip operation apparatus according to claim 14, wherein the second gripper comprises:
clip holding parts formed at left and right ends thereof to hold left and right sides of the hemostatic clip, and
clip bending parts formed at upper and lower ends thereof to press and bend upper and lower sides of the hemostatic clip.
